# EUROPEAN PATENT APPLICATION

(11) **EP 3 533 332 A1**
(43) Date of publication of application: **04.09.2019**
(21) Application number: 18159610.7
(22) Date of filing: 01.03.2018
(51) Int. Cl.: A01N 63/00, A01N 63/02, A01N 63/04, A01N 25/22, A01N 25/28, A01P 7/00

(54) **UV RESISTANT BIOPESTICIDE MICROPARTICLES**

(71) Applicant: Exosect Limited, Colden Common Winchester Hampshire SO21 1TH (GB)
(72) Inventor: BRAUN, Martin, Hampshire, GU31 4AP (GB); CURCIC, Igor, Hampshire, SO21 1TH (GB); GRZYWACZ, David, Kent, ME13 7NB (GB); WILSON, Kenneth, Lancashire LA2 8NF (GB)
(74) Representative: Boynton, Juliette Alice

(57) **Abstract**

A delivery method for bacteria, viruses, fungi and dsRNA in which the bacteria, virus, fungus or dsRNA is protected from degradation by UV light by encapsulation in a particle of wax also containing a UV blocker. The formulation may also include other chemistry, such as insecticides. The particles are edible by insects and their larva and result in ingestion of the bacteria, virus, fungi and/or dsRNA by the insect or larva, resulting in control thereof.

## Description

### Introduction

The present invention relates to composite particles for the delivery of biological agents such as bacteria, viruses, fungi and dsRNA to plants and animals for use in the control of pest infestations, in particular insect pests, and to particles for use in such delivery.

Global biopesticide sales have increased from around US$0.6 billion in 2003 to around US$ 3 billion today and are projected to reach US$11 billion by 2025. With a Compound Annual Growth Rate (CAGR) of 16-17%, biopesticides are currently the fastest growing sector in the crop protection market sector.

The biopesticide market currently accounts for 3% of the overall crop protection market. Virus based biopesticides are estimated to be less than 10% of this, with significant potential for growth.

Certain entomopathogenic biological agents such as entomopathogenic bacteria, viruses, fungi and dsRNA are known that are effective in the control of plant and animal pests. However, delivery of such biological agents to such pests is problematic as all are very susceptible to degradation by UV, such as in sunlight. Typically, such biological agents will last in the field for a small number of days, for example 2 - 7 days. Current delivery formulations include the bacteria, virus, fungi and/or dsRNA in a liquid formulation in which the bacteria, virus, fungus and/or dsRNA are suspended, and including a UV blocker dissolved in the liquid. However, on delivery to the plant or animal, the bacteria, virus, fungus and/or dsRNA is separated from the UV blocker, with very little of the UV blocker in the vicinity of the bacteria, virus, fungus or dsRNA. Encapsulation techniques have also been attempted using polymeric substances with titanium dioxide or carbon black as a light absorber. These techniques have also failed to protect the biological agents from UV degradation. As a result, the use of bacteria, viruses, fungi and dsRNA to control pests in plants and animals is very limited.

A particular problem has been found with GM crops that have been designed to cause mortality in pests consuming the crops. Pests have started to develop resistance to such crops. One solution would be to treat such pests with bacteria, viruses, fungi and dsRNA. However, formulations currently available degrade in UV light and thus do not remain active for a suitable period of time. One advantage of the use of bacteria, viruses, fungi and dsRNA is that they tend to have a narrow host insect range, and infectivity is restricted to a host genus or family. Generally, these biological agents have the additional advantage of being substantially non-toxic to vertebrates such as mammals, including humans, and are generally not known to be toxic to beneficial insects, birds or to aquatic organisms.

A further problem associated with conventional commercial formulations is that the virus of choice is typically suspended in naked form in an aqueous formulation which contributes to limiting or reducing the shelf-life of the virus through the action of oxidative processes.

There is currently no effective, economically viable, commercial solution to improve the resilience of biopesticides to UV.

There therefore exists a need to provide alternative and preferably improved UV-stable formulations for delivery of biological agents such as bacteria, viruses, fungi and/or dsRNA to plants and animals. In addition, there exists a further need to provide formulations in which the biological agent e.g. bacteria, virus, fungi and/or dsRNA is less susceptible to oxidation.

### The Invention

According to a first aspect of the invention there is provided a composite particle having a volume median diameter in the range 2 - 200 microns comprising:
i. at least one biological agent selected from a bacteria, virus, a fungus, a nucleic acid and combinations of two or three thereof;
ii. at least one UV blocker; and
iii. wax.

Such particles can be applied to, and will adhere to, plants and animals. Any insects infesting the plant or animal may come into contact with, and typically eat the particle, becoming infected with the bacteria, virus, fungus and/or nucleic acid, e.g. dsRNA, which will lead to the control of the insect infestation.

Preferably the wax will be one that is degradable and/or soluble in the gut of an insect or larva thereof. Suitable waxes include those that solubilise or degrade or surface etch enough to release at least one viable virus particle in a larval gut environment, for example, in a basic pH. Such waxes are typically selected from natural waxes that are ingestible by larvae, such as carnauba wax, rice bran wax, candelilla wax, sugar cane wax, ouricouri wax. Other suitable waxes include synthetic waxes, such as amide waxes, polyethylene waxes, functionalised polyethylene waxes, oxidised polyethylene waxes or a mixture or blend of two or more thereof. Preferred waxes include carnauba wax, beeswax, rice bran wax and candelilla wax and mixtures thereof. Typically, waxes of use in the invention have a melting temperature of ≥40°C, depending on design. Suitable natural waxes of use in the invention include waxes having a melting point of preferably ≥50°C, and most preferably are made up of hard waxes having a melting point of ≥70°C. Examples of natural waxes of use in the present invention include carnauba wax, beeswax, Chinese wax, shellac wax, spermaceti wax, myricyl palmitate, cetyl palmitate, candelilla wax, castor wax, ouricury wax, wool wax, sugar cane wax, retamo wax, rice bran wax and the like.

The UV blocker may be any agent capable of blocking the adverse effects of UV light, and for example may be titanium dioxide, oxyl methoyxcinnimate, modified soya oil, zinc oxide nano-particles, beta-carotene, p-amino benzoic acid, stilbene type UV protectants (e.g. resveratrol), and mixtures thereof.

Particles of the invention typically contain UV blocker in an amount of up to 20% by weight of the wax, preferably up to 10% by weight, preferably at least 0.5%, preferably at least 1%. The % can vary according to blocker potency. In specific examples we used TiO₂ at about 5% w/w (50g TiO₂ / 1 Kg), Blankophor PO1 at about 2% w/w (20g/Kg), Blankophor DSA at about 2% (20g/Kg) and Blankophor DS at about 2% w/w (20g/Kg).

Typically, the particle may further comprise a chemical active agent. Preferably the chemical active agent is an insecticide. Alternatively, the chemical agent may be a insect attractant or a semiochemical. Suitable insecticides include a pyrethroid or an organophosphate insecticide. Suitable insecticides that may be added to composite particles of the invention include α-cypermethrin, A-cyhalothrin, [cyano-(3-phenoxyphenyl)-methyl]-3-(2,2-dibromoethenyl)-2,2dimethylcyclopropane-1-carboxylate (deltamethrin), T-fluvalinate, pirimiphos methyl, chlorpyriphos, malathion, terbufos, phosmet, tiamethoxam, clothiandin, acetamiprid, spinosad, rynaxapyr and the like.

Conveniently, the particle may further comprise an added feed stimulant such as sugars, for example, sucrose, fructose, palm sugar, golden cane syrup and the like admixed therein in particulate or liquid form, molasses, honey, sorbitol or other artificial or organic baits, including volatiles selected from alcohols, esters and aromatic compounds, such as ethyl acetate, 3-methylbutanol, ethyl hexanoate, 2-phenylethanol, ethyl octanoate, ethyl (E)-4-decenoate, ethyl decanoate, ethyl dodecanoate and the like, or other plant extracts or a larvae attractant, such as a larvae aggregation pheromone, for example that of *Cydia pomonella.*

The particles may include other additives such as colouring agents such as optical brighteners and commercially available colouring agents such as food colouring agents, plasticisers such as glycerine or soy oil, antioxidants such as vitamin E, butylated hydroxyl anisole (BHA), butylated hydroxytoluene (BHT), and other antioxidants that may be present, or mixtures thereof.

The biological agent may comprise any bacteria effective in controlling insects, in particular insects that infest GM (genetically modified) crops and are resistant to those GM crops. Suitable bacteria include Bacillus thuringiensis, Lysinibacillus sphaericus and Paenibacillus popilliae.

A preferred bacterium is Bacillus thuringiensis. Bacillus thuringiensis (or Bt) is a Gram-positive, soil-dwelling bacterium, commonly used as a biological pesticide. Bt also occurs naturally in the gut of caterpillars of various types of moth and butterflies, as well as on leaf surfaces, aquatic environments, animals faeces, insect-rich environments, and flour mills and grain-storage facilities. It has also been observed to parasitize other moths such as Cadra calidella, in laboratory experiment working with C. calidella, many of the moths were diseased due to this parasite.

Suitable viruses for inclusion in the biological agent include baculoviruses and granulosis viruses. Suitable fungi include entomopathogenic fungi (e.g. Beauveria species and Hirsutella species).

Nucleic acids in general are suitable for use as biological agents in the invention. The nucleic acids may be DNA or RNA, and may be single or double stranded (ss or ds). In particular, the particles may contain RNA for use in RNAi approaches to pest control - see for example the review by Mamta, B. & Rajam, M.V. Physiol Mol Biol Plants (2017) 23: 487.

Preferred biological agents are bacteria, fungi and nucleic acids, especially bacteria and nucleic acids, especially RNA.

Particles of the invention typically contain the biological agent in an amount of up to 20% by weight of the wax, preferably up to 10% by weight, preferably at least 0.1%, preferably at least 0.5%. The % can vary according to agent potency.

Particles comprising wax are known to easily acquire an electrostatic charge, which enables them to adhere to plants and animals. Particles of any size (conveniently measured as volume median diameter, VMD) up to 200 µm, and preferably up to 100 µm are suitable for use in the invention, including for adherence to plants and animals. In preferred embodiments the particles are of a size suitable for them to be eaten by an insect or larva thereof. Preferably the particles have a VMD of 2 - 200 µm, more preferably the particles have a VMD of 5 - 100 µm, and most preferably the particles have a VMD of 5 - 50 µm or 5 - 50 µm. Particles used in examples had VMDs of approximately 10-15 µm.

Arthropod species which may be targeted by composite particles of the invention include crop pest species of the Lepidoptera, pest species of the Diptera, and pest species of the Coleoptera such as of the Scarabaeidae. The insect pests that may be targeted using composite particles of the invention are typically members of the Lepidoptera and include the larvae of Lepidoptera species that infest food processing and food storage sites, such as Tobacco moth also known as Warehouse moth (*Ephestia elutella*)*,* Mediterranean Flour moth (*Ephestia Kuehniella*) [also known as 'Indian Flour moth' and 'Mill moth'], Raisin moth (*Cadra figulilella*), Almond Moth (*Cadra cautella*) and Indian Meal moth (*Plodia interpunctella*)*.* Other insect pests that infest growing crops which may be targeted using composite particles of the invention include the larvae of Corn earworm also known as the tomato fruitworm or Tobacco budworm [*Helicoverpa zea*]*,* Cotton bollworm, Podborer [*Helicoverpa armigera*], Beet armyworm [*Spodoptera exigua*], Egyptian cotton leafworm [*Spodoptera littoralis*], African armyworm *Spodoptera exempta,* Velvetbean caterpillar [*Anticarsia gemmatalis*]*,* Gypsy moth [*Lymantria dispar*], Codling moth [*Cydia pomonella*], Diamond back moth [*Plutella xylostella*], False Codling moth [*Thaumatotibia leucotreta*],Potato tuber moth [*Phthorimaea operculella*], Summer fruit tortrix moth [*Adoxphyes orana*], Oriental tea tortrix moth [*Homona magnanima*], and Smaller tea tortrix moth, [*Adoxophyes honmai*]*.*

Preferably the particles are prepared by a method comprising:
i) melting at least one wax;
ii) adding at least one UV blocker to the molten wax of i);
iii) adding at least one of bacteria, viruses, fungi and dsRNA to the molten wax of ii) and admixing therewith;
iv) cooling the product of iii) to a solid; and
v) kibbling and comminuting the product of iv) to a particle size as defined elsewhere.

In step i), the wax may be a single wax or a mixture of waxes. Other additives, such as chemical additives, bait or feeding stimulants may also be added in step ii). The cooling step iv) is preferably rapidly achieved. For example, this may be by pouring the liquid admixture of iii) into a large shallow tray or other suitable receptacle that is then placed inside a suitable freezing means, such as a freezer, and held at a temperature in the range of about minus 5°C to minus 30°C, such as at minus 24°C. Further means to attain rapid melting in i) and cooling of the product of iii) in step iv) include the use of commercially available mantle vessels, such as the Style D kettle with super jacket available from Lee Industries, Philipsburg, USA, that are capable of heating admixtures of the invention to high liquefying temperatures and then rapidly cooling them to an intermediate temperature, such as the melting temperature of the blend, prior to pouring the cooled liquid into a receptacle such as a tray and refrigerating further, forming a solid body of wax. Lastly, the order of adding the UV blocker and biological agent is not material, the order can be reversed or they can be added at the same time.

Once the cooling step of iv) is completed, the resultant block of wax may then be kibbled, comminuted and micronized to particle sizes of the desired diameter.

Bacteria, viruses, fungi and nucleic acids may be temperature sensitive and may not be able to withstand the melting temperature of the wax. Where this is the case, other methods of preparation of the particles are known, such as particles from gas saturated solutions. In this and other methods, the use of high pressure can lower the melting temperature of the wax, thus enabling the viability of the biological agent to be retained.

The particles of the first aspect may be used in a method of delivery of the biological agent to plants or animals for treatment of an insect infestation. The treatment may be a prophylactic treatment. The use may be on a GM plant to which the insects have developed resistance.

The particles of the first aspect may also be of use in cosmetics formulations, bioremediation, and food manufacturing.

Preferred compositions of the invention are those comprising a plurality of particles according to any preceding claim.

In further aspects, the invention provides a composite particle or a composition according to the invention, for use in control of an insect infestation of a plant or animal.

In further aspects, the invention provides a composite particle or a composition for use according to the invention, for control of an insect infestation of a GM plant, e.g. control of infestation by insects that are resistant to the GM plant.

The composite particle or composition is dry in preferred embodiments.

In further preferred embodiments, the composite particle or composition is in the form of a liquid suspension and is e.g. suitable for spray application.

According to a second aspect of the invention there is provide dry composite particles for use in the control of an insect infestation of a plant or animal, wherein the particles comprise:
i. at least one biological agent selected from a bacteria, a virus, a fungus and a dsRNA;
ii. at least one UV blocker; and
iii. wax,
the particle formed such that the UV blocker is distributed throughout the wax, and the bacteria, virus, fungus and/or dsRNA is fully encapsulated within the wax and UV blocker.

According to a third aspect of the invention there is provided a liquid formulation comprising particles suspended in a liquid for use in the control of an insect infestation of a plant or animal, wherein the particles comprise
iv. at least one biological agent selected from a bacteria, a virus, a fungus and a dsRNA;
v. at least one UV blocker; and
vi. wax,
the particle formed such that the UV blocker is distributed throughout the wax, and the bacteria, virus, fungus and/or dsRNA is fully encapsulated within the wax and UV blocker.

In respect of the second and third aspects of the invention, the composite particles are as described in respect of the first aspect.

The liquid formulation may be either an aqueous liquid or an oleaginous liquid formulation.

Aqueous formulations may include surfactants selected from commercially available ranges of surfactants sold under the trade marks such as Libsorb, Silwet, Tween, Span, Tensiofix, Brij, Torpedo, Newmans, Lansurf, Atplus, Atlox, Synperonic, Fortune, Guard, Rhino, Biopower, and the like. Of these surfactants, Tensiofix, and Span are most preferred.

Liquid formulations of this aspect of the invention may include additional feeding stimulants in liquid or solid form, as described hereinabove.

The liquid formulations according to this aspect of the invention may be applied to eukaryotic tissue selected from plant tissue, such as leaves, stems, fruiting bodies, and flowers.

Oleaginous formulations, that is to say oil dispersion (OD) formulations, may contain any oil suitable for use in the invention which may be selected from petroleum oils, such as paraffin oil, summer spray oils, the solvesso® range of solvents, petroleum spirits and winter spray oils known in the art, and vegetable oils such as rapeseed oil, soybean oil, sunflower oil, palm oil and the like.

Composite particles of the invention once delivered to target surfaces are capable of adhering thereto, as the aqueous element of the composition evaporates or, in the case of an oleaginous element, the oil disperses and are available for ingestion by the feeding target larvae.

### Advantages

An advantage of formulations of the invention is improved UV protection of active biological agents, e.g. bacteria and/or nucleic acids, for pesticide use. Inclusion in formulated wax particles protected active agent from UV degradation during field application and may assist in reducing oxidative stress during storage. It has been found that agents were protected by the formulations on the leaf surface, and become activated in the alkaline conditions of the insect mid-gut.

A further advantage is that wax-containing particles of the invention can be co-formulated with adjuvants that are known to absorb or reflect UV and improve efficacy without the need for UV stabilisers in tank mixes in the field - this latter approach shown to not be cost effective, due to the high rates needed to show positive effects. Also tank mixing as per the prior art may be needed in such quantities with particulate additives that sprayability is adversely affected, another disadvantage that can be avoided in the present invention.

Particles of the invention, incorporating UV blockers adjuvants within the active agent-virus loaded particles, improved UV protection and greatly reduced the amounts needed, therefore reducing formulation cost.

There now follow examples that illustrate the invention. It is to be understood that the examples are not to be construed as limiting the invention in any way.

### Examples

### INTRODUCTION

Biological agent (virus) was formulated in wax particles comprising UV blocker.

### Key:

BV: Baculovirus Occlusion Bodies raw material
NPV: Nucleopolyhedrovirus, a genera of the baculoviridae family of viruses included under the BV umbrella
GV: Granulovirus, a genera of the baculoviridae family of viruses included under the BV umbrella

### 1. MATERIALS:

Active ingredient (BV of the chosen species - SpliNPV targeting *Spodoptera littoralis,* or the Egyptian cotton leaf worm) sourced from NRI Candelilla wax and Rice bran wax.
Titanium dioxide (TiO₂)

### 2. EQUIPMENT:

Hotplate that heats up to 150°C (Stuart scientific Ltd)
Two decimal place balance (Ohaus®)
Freezer that cools down to at least -24°C (any make/ model will do)
High shear mixer/ homogenizer (IKA® T18 digital)
Kibbler mill (KT handling limited model 04)
Comminuting mill (Apex® LTD type 314s)
Air jet mill (any make/ model will do)
Suitable size sample pots

### 3. DETAILED PROCEDURE

3.1. Using a calibrated balance weigh out the required quantity of carrier waxes - 125.0g of rice bran wax, 125.0g of candelilla wax and 12.50g of TiO₂.
   Place a 50:50 mixture candelilla wax: rice bran wax (% w/w) into a pan and place onto a hotplate set to a temperature of 120°C. The wax is heated until completely melted and a clear liquid with no solids is observed.
   Using a calibrated balance a quantity of the active BV at 1% w/w is weighed out.
3.1.1. The BV and TiO₂ are added together to the molten wax quickly over a total time period of 60s and dispersed within the molten wax by high shear mixing (using a high shear mixer IKA® T18) to ensure even distribution within the wax.
3.2. The wax is then transferred to a foil lined shallow tray and transferred to a freezer set at -24°C to rapidly cool and solidify within approximately 1-2h.
3.3. Once the formulated material is frozen into a completely solid block or slab it is broken into large chunks and sent for milling. The chunks are ground in a kibbler mill (KT Handling Limited, Model 04) to particles of approximately 2mm average diameter
3.4. The kibbled material is then comminuted into smaller particles in a comminuting mill (model 314s, from Apex® Ltd) to particles of 150µm average diameter.
3.5. The comminuted material is further micronized in a jet mill (Hosokawa® Alpine Jet AFG 100 fluidised bed jet mill) to achieve granulation of particle size ∼10um.
3.6. The micronized material containing baculovirus particles and TiO₂ is stored in a suitable sample container under refrigeration conditions at 4°C, until use.

The particles were used for testing pest mortality rates against controls.

### Key Findings

Study results showed consistent protection of baculovirus biopesticide from UV radiation in the formulation:

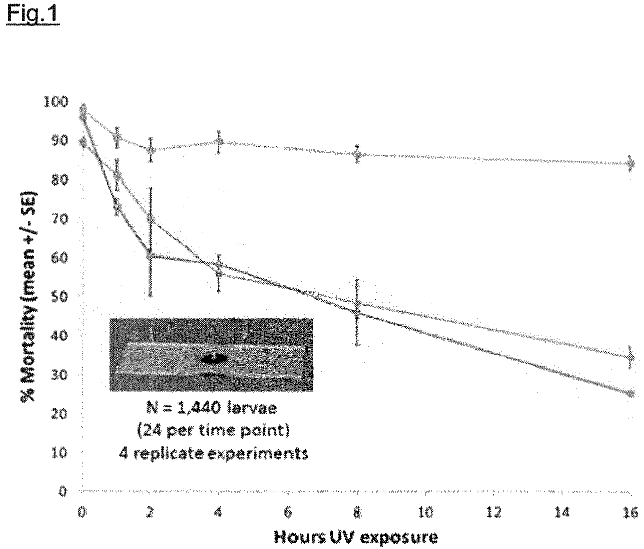

Replicated bioassays showed a consistent effect of the UV blocker-containing formulation (top line), with >80% efficacy being retained after 16 hours UV exposure (equivalent to 5 days exposure to sunlight in the field), compared to around 30% for the raw NPV virus (middle line) or commercial biopesticide product (lower line). A further bioassay indicated that >80% efficacy was retained even after 96 hours UV exposure (equivalent to around 30 days in the field).

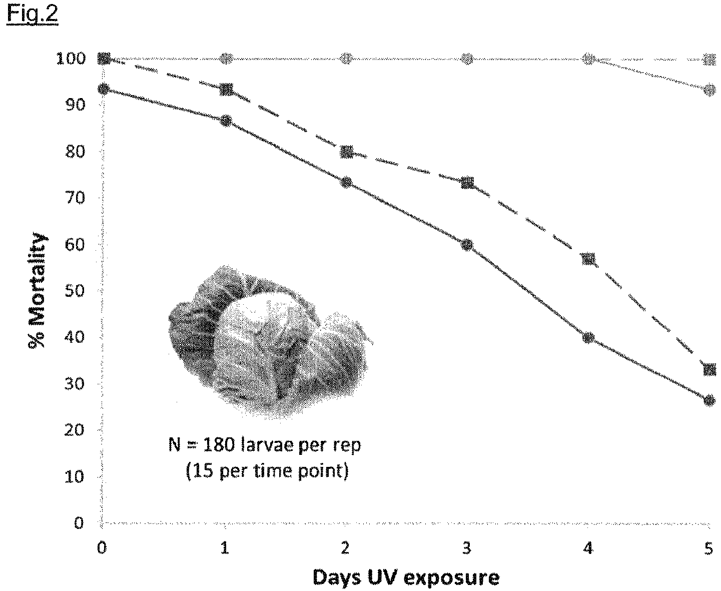

Spraying of a single dose of raw NPV, the commercial standard or formulations of the invention onto tomato or cabbage plants, then exposing them to UV lights simulating sunlight for 12 hours per day for 0,1,2,3,4 or 5 days showed the two formulations of the invention (top two lines) gave >90% efficacy after 5 days, compared to the commercial standard (lower two lines) which gave around 30%.

### Tomato Plant Trial

A further example was carried out comparing a formulation of the invention against a commercially available product (Littovir (SpliNPV). Data were obtained for mortality up to 8 days post-infection and showed mortality retained at 60% after 8 days exposure to UV compared with 10% for Littovir (which after 8 days had mortality reduced to that of the control, namely water).

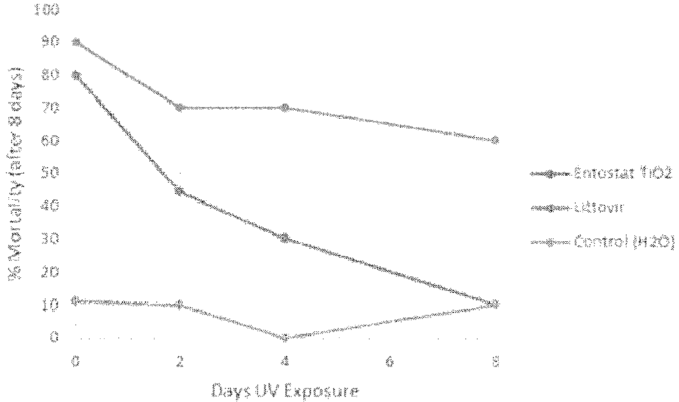

Subsequent analysis showed that loss of activity for the formulations of the invention occurred within the first hour of UV exposure. This may be due to degradation of any non-encapsulated virus present; in which case, protection conferred by the formulation may have retained the virus with essentially no degradation as a result of UV exposure.

### Discussion

One of the limitations of the use of biological agents, e.g. baculoviruses, as biological control agents is their loss of activity under field conditions due to inactivation by ultraviolet light.

When used in the field, the formulations of the invention can extend and even dramatically extend the field life of active agents, e.g. biopesticides, meaning that farmers can reduce the number of sprays required to protect the crops and/or that biopesticide producers can drastically reduce the amount of active ingredient to achieve the same result. Prophylactic spraying of the biopesticide is now made an option because of the longevity achieved by the invention. The examples used virus but the invention is of application to other UV sensitive biological agents.

UV stable formulations of the invention can also increase the plant range and growing conditions for virus biopesticide applications.

## Claims

1. A composite particle having a volume median diameter in the range 2 - 200 microns comprising:
i. at least one biological agent selected from a bacteria, a fungus, a nucleic acid and combinations of two or three thereof;
ii. at least one UV blocker; and
iii. wax.

2. A composite particle according to claim 1, wherein the UV blocker is distributed throughout the wax.

3. A composite particle according to claim 1 or 2, wherein the UV blocker is present in an amount up to 20% by weight of the particle.

4. A composite particle according to any preceding claim, wherein the biological agent is present in an amount up to 20% by weight of the particle.

5. A composite particle according to any preceding claim, wherein the nucleic acid comprises dsRNAi.

6. A composite particle according to any preceding claim, wherein the biological agent comprises bacteria.

7. A composite particle according to claim 6, wherein the bacteria is *Bacillus thuringiensis.*

8. A composite particle according to any preceding claim, wherein the wax is selected from natural waxes, synthetic waxes, mineral waxes and mixtures thereof.

9. A composite particle according to any preceding claim, further comprising a chemical active agent.

10. A composite particle according to any preceding claim, having a volume median diameter in the range of 5 - 100 microns.

11. A composition comprising a plurality of particles according to any preceding claim.

12. A composite particle or a composition according to any preceding claim, for use in control of an insect infestation of a plant or animal.

13. A composite particle or a composition for use according to claim 12, for control of an insect infestation of a GM plant, e.g. control of infestation by insects that are resistant to the GM plant.

14. A composite particle or a composition for use according to claim 12 or 13, wherein the composite particle or composition is dry.

15. A composite particle or a composition for use according to claim 12 or 13, wherein the composite particle or composition is in the form of a liquid suspension.
